(19) **Europäisches Patentamt** · **European Patent Office** · **Office européen des brevets**

(11) **EP 3 584 287 B1**

(12) **EUROPEAN PATENT SPECIFICATION**

(45) Date of publication and mention
of the grant of the patent:
**13.07.2022 Bulletin 2022/28**

(21) Application number: **18754381.4**

(22) Date of filing: **08.02.2018**

(51) International Patent Classification (IPC):
**C08L 101/14** (2006.01)   **A61F 13/15** (2006.01)
**A61F 13/53** (2006.01)   **A61L 15/20** (2006.01)
**A61L 15/46** (2006.01)   **A61L 15/50** (2006.01)
**C08K 5/32** (2006.01)   **C08J 3/12** (2006.01)
**C08L 33/02** (2006.01)   **C08L 33/12** (2006.01)
**C08K 5/13** (2006.01)   **C08K 5/09** (2006.01)

(52) Cooperative Patent Classification (CPC):
(C-Sets available)
**C08K 11/00; A61F 13/53; A61L 15/40; A61L 15/46;
C08L 101/14;** A61L 2300/30; C08L 33/02   (Cont.)

(86) International application number:
**PCT/JP2018/004324**

(87) International publication number:
**WO 2018/150992 (23.08.2018 Gazette 2018/34)**

(54) **WATER-ABSORBING RESIN COMPOSITION, ABSORBENT BODY, AND ABSORBENT ARTICLE**

WASSERABSORBIERENDE HARZZUSAMMENSETZUNG, SAUGFÄHIGER KÖRPER UND SAUGFÄHIGER ARTIKEL

COMPOSITION DE RÉSINE ABSORBANT L'EAU, CORPS ABSORBANT ET ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.02.2017 JP 2017025739**

(43) Date of publication of application:
**25.12.2019 Bulletin 2019/52**

(73) Proprietor: **Sumitomo Seika Chemicals Co., Ltd.
Kako-gun, Hyogo 675-0145 (JP)**

(72) Inventors:
• **TANIMOTO, Daiki**
Himeji-shi
Hyogo 672-8076 (JP)
• **MURAKAMI, Masahiro**
Himeji-shi
Hyogo 672-8076 (JP)

(74) Representative: **Mewburn Ellis LLP
Aurora Building
Counterslip
Bristol BS1 6BX (GB)**

(56) References cited:
CN-A- 104 173 591    JP-A- H03 261 479
JP-A- 2003 286 165    JP-A- 2004 156 010
JP-A- 2004 210 924    JP-A- 2005 029 751
JP-A- 2005 198 854    JP-A- 2010 540 685
KR-A- 20100 039 532

• Oryza Oil & Fat Chemical Co. ET AL: "EVENING PRIMROSE EXTRACT For Anti-Diabetes & Anti-Obesity", , 19 October 2007 (2007-10-19), XP055733382, Retrieved from the Internet: URL:http://www.oryza.co.jp/html/english/pd f/tukimi-e4.1.pdf [retrieved on 2020-09-23]
• Pharcos Co. ET AL: "Lunawhite (Evening Primrose Seed Extract)", , 12 December 2005 (2005-12-12), page 32, XP055733351, Retrieved from the Internet: URL:http://miyoshiamerica.com/natural-extr acts/ [retrieved on 2020-09-23]

• Ioana Roman ET AL: "CITOTOXIC EFFECTS OF THREE SPECIES OF EPILOBIUM (ONAGRACEAE) HERBAL EXTRACTS IN RATS", Studia Universitatis "Vasile Goldis". Seria stiintele vietii, 1 January 2010 (2010-01-01), pages 19-23, XP055733487, Arad Retrieved from the Internet: URL:https://search.proquest.com/docview/231812998?pq-origsite=gscholar&fromopenview=true

(52) Cooperative Patent Classification (CPC): (Cont.)

C-Sets
**C08K 11/00, C08L 33/02**

**Description**

Technical Field

The present invention relates to a water-absorbent resin composition, an absorbent material, and an absorbent article; more particularly, the present invention relates to a water-absorbent resin composition that constitutes an absorbent material suitably used for hygienic materials, such as disposable diapers, sanitary napkins, and incontinence pads, and to an absorbent article obtained using the absorbent material.

Background Art

In recent years, water-absorbent resins have been widely used in the field of hygienic materials, such as disposable diapers, sanitary napkins, and incontinence pads.

As such water-absorbent resins, crosslinked products of acrylic acid partially neutralized salt polymers have been proposed as preferable water-absorbent resins, because they have a number of advantages in that, for example, they have good water-absorption capacity; acrylic acid used as a raw material is readily industrially available, and thus, they can be produced at low cost with uniform quality; and they are resistant to decomposition or degradation.

On the other hand, an absorbent article such as a disposable diaper, a sanitary napkin, or an incontinence pad is composed of an absorbent material that absorbs and retains a body fluid such as urine or menses excreted from the body, the absorbent material being positioned mainly in a central portion, a liquid-permeable front sheet (top sheet) positioned on the side that is brought into contact with the body, and a liquid-impermeable rear sheet (back sheet) positioned opposite to the side that is brought into contact with the body. The absorbent material is composed of hydrophilic fibers such as pulp, and a water-absorbent resin.

This absorbent material, when used in a hygienic material, for example, may generate an unpleasant odor such as ammonia, as a result of absorbing a body fluid, particularly urine, blood, sweat, or the like.

As techniques for inhibiting such an unpleasant odor, the following methods are known, for example: a method in which an odorous component adsorbent such as activated carbon or zeolite is mixed into an absorbent material (see Patent Literatures 1 and 2, for example); a so-called masking method in which a fragrance stronger than an unpleasant odor component is generated by an absorbent material (see Patent Literature 3, for example); a method in which a component that causes an unpleasant odor is chemically modified into a component that does not generate the unpleasant odor; and a method in which a heavy metal is mixed into an absorbent material to kill bacteria that generate an unpleasant odor (see Patent Literature 4, for example).

On the other hand, in the production of an absorbent material using a water-absorbent resin, low flowability of the water-absorbent resin may cause the productivity to decrease. For example, depending on the component added when adopting a technique for inhibiting an unpleasant odor, the flowability of the water-absorbent resin may further decrease.

CN 104173591 A concerns a Puer tea essential oil hydrogel patch comprising a carrier and a traditional Chinese medicine ointment. KR 20100039532 A concerns a hydrogel composition for treating atopy contains Pseudoceramide, cerix 5, cerasol, moistin, morus bark extract, Oenothera odorata seed oil, jojoba oil, olive oil, Elhibin or Stimutex AS first raw material, Scutellaria baicallensis Georgi extract, Pleuropterus mulflorus extract, chamomile extract, or dismutin BT second raw material, Phytospingosine, Atofree, or Lipacide UG third raw ingredient.

Citation List

Patent Literature

    Patent Literature 1: JP 2001-37805 A
    Patent Literature 2: JP H11-512946 A
    Patent Literature 3: JP H6-51843 B
    Patent Literature 4: JP 2001-505237 A

Summary of Invention

Technical Problem

A main object of the present invention is to provide a water-absorbent resin composition that can inhibit an unpleasant odor, and has excellent flowability.

Solution to Problem

The present inventors conducted extensive research to solve the above-described problem. As a result, the inventors have found that a water-absorbent resin composition containing an *Onagraceae* family plant extract and a water-absorbent resin can inhibit an unpleasant odor, and unexpectedly, the composition exhibits excellent flowability.

The present invention has been accomplished as a result of further research based on this finding.

In summary, the present invention provides aspects of invention comprising the following features:

Item 1. A water-absorbent resin composition comprising an *Onagraceae* family plant extract and a water-absorbent resin, wherein

the *Onagraceae* family plant extract is contained in an amount of 0.05 parts by mass or more per 100 parts by mass of the water-absorbent resin,
the *Onagraceae* family plant extract is an *Oenothera* genus plant extract,
the *Onagraceae* family plant extract is obtained from seeds using water or hydrous ethanol,
the water-absorbent resin is in the form of particles, wherein the water-absorbent resin composition is produced by mixing the *Onagraceae* family plant extract and the water-absorbent resin.

Item 2. The water-absorbent resin composition according to item 1, wherein the *Onagraceae* family plant extract is contained in an amount of 0.05 to 5 parts by mass per 100 parts by mass of the water-absorbent resin.

Item 3. An absorbent material comprising the water-absorbent resin composition according to item 1 or 2, and a hydrophilic fiber.

Item 4. An absorbent article comprising the absorbent material according to item 3, the absorbent material being held between a liquid-permeable sheet and a liquid-impermeable sheet.

Advantageous Effects of Invention

In accordance with the present invention, there is provided a water-absorbent resin composition that can inhibit an unpleasant odor, and has excellent flowability. Furthermore, in accordance with the present invention, there is provided an absorbent article obtained using the water-absorbent resin.

Description of Embodiments

1. Water-Absorbent Resin Composition

A water-absorbent resin composition according to the present invention comprises an *Onagraceae* family plant extract and a water-absorbent resin. Because the water-absorbent resin composition comprises the *Onagraceae* family plant extract, even if the composition absorbs a component that causes an unpleasant odor, it can effectively inhibit the generation of the unpleasant odor (in particular, ammonia, which is a representative component that causes an unpleasant odor). Furthermore, because the water-absorbent resin composition comprises the *Onagraceae* family plant extract, it unexpectedly exhibits improved flowability, which can increase the productivity of an absorbent material or the like obtained using the water-absorbent resin composition. The water-absorbent resin composition of the present invention will be hereinafter described in detail.

(*Onagraceae* Family Plant Extract)

The *Onagraceae* family plant extract contained in the water-absorbent resin composition of the present invention is an extract of a plant in the family *Onagraceae*. The plant in the family *Onagraceae* is a plant in the genus *Oenothera*. Specific examples of the plant in the genus *Oenothera* include *Oenothera tetraptera*, *Oenothera laciniata*, *Oenothera stricta*, *Oenothera biennis*, *Oenothera erythrosepala*, and *Oenothera speciosa*. The *Onagraceae* family plant extracts may be used alone or in combinations of two or more. In particular, *Oenothera tetraptera*, *Oenothera laciniata*, *Oenothera stricta*, *Oenothera biennis*, *Oenothera erythrosepala*, and *Oenothera speciosa* in the genus *Oenothera* are plants that are very similar to one another; therefore, an extract of only one of these plants can be suitably used, or extracts of two or more plants can be suitably used in combination.

As the *Onagraceae* family plant extract, an extract obtained from seeds, leaves, stalks, roots, flowers, or the like of a plant in the family *Onagraceae* can be used. From the viewpoint of further improving the unpleasant odor-inhibiting effect and the flowability of the water-absorbent resin composition, an extract obtained from seeds of a plant in the family *Onagraceae* is preferred among these extracts. A seed of a plant in the family *Onagraceae* includes the seed coat, endosperm, hypocotyl, cotyledon, and the like.

The solvent to be used in the preparation of the *Onagraceae* family plant extract is water or hydrous ethanol. The extraction temperature may be a temperature from 0°C to approximately the boiling point of the solvent, for example, although not particularly limited thereto. The extraction time may be from 30 minutes to 3 hours. In the preparation of the *Onagraceae* family plant extract, a method of multi-stage extraction using any of these solvents may be adopted.

As the *Onagraceae* family plant extract, a commercially available product can be readily used, and examples of such products include trade name "Evening Primrose Extract-P, *Oenothera biennis* extract content 100%" and trade name "Evening Primrose Extract-WSPS, *Oenothera biennis* extract content 100%" from Oryza Oil & Fat Chemical Co., Ltd.; and trade name "LUNAWHITE B, *Oenothera biennis* extract content 1% solution" from Ichimaru Pharcos Co., Ltd.

In the present invention, an *Onagraceae* family plant extract obtained by the above-described extraction method may be used as is, as long as the effects of the present invention can be achieved. The extract may also undergo separation and purification, as required, and the resulting extract with increased purity may be used. As the purification method, any method appropriately selected from conventionally known methods can be adopted, and examples of such methods include, but are not particularly limited to, organic solvent precipitation, centrifugation, ultrafiltration, high performance liquid chromatography, and column chromatography. Furthermore, as the *Onagraceae* family plant extract of the present invention, an extract obtained by the above-described extraction method may be used as a liquid, or the extract may undergo a treatment such as drying under reduced pressure, freeze-drying, or the like, as required, and the resulting material may be used as a powder.

In the water-absorbent resin composition of the present invention, the blending ratio of the *Onagraceae* family plant extract to the water-absorbent resin is set out as follows. From the viewpoint of further improving the unpleasant odor-inhibiting effect and the flowability of the water-absorbent resin composition, while ensuring sufficient water-absorption capacity, the lower limit of the amount of the *Onagraceae* family plant extract contained per 100 parts by mass of the water-absorbent resin is 0.05 part by mass or more. Furthermore, from the viewpoint of easy industrial production and costs, the upper limit of the amount of the *Onagraceae* family plant extract contained per 100 parts by mass of the water-absorbent resin is preferably 5 parts by mass or less, more preferably 2.5 parts by mass or less, and still more preferably 1.0 part by mass or less. These lower limit values and upper limit values can be combined as desired, and the amount of the *Onagraceae* family plant extract contained per 100 parts by mass of the water-absorbent resin is preferably 0.05 to 5 parts by mass (0.05 part by mass or more and 5 parts by mass or less), 0.05 to 2.5 parts by mass, or 0.05 to 1.0 part by mass.

The water-absorbent resin composition of the present invention is readily produced by mixing the *Onagraceae* family plant extract and the water-absorbent resin.

In order for the water-absorbent resin composition of the present invention to exhibit an excellent unpleasant odor-inhibiting effect and excellent flowability, the *Onagraceae* family plant extract may be added, either as a powder or a liquid, to the water-absorbent resin.

(Water-Absorbent Resin)

The water-absorbent resin contained in the water-absorbent resin composition of the present invention is composed of, for example, a polymer of a water-soluble ethylenically unsaturated monomer.

The water-absorbent resin is in the form of particles. The water-absorbent resin preferably has a median particle diameter of 200 to 600 $\mu$m, more preferably 250 to 500 $\mu$m, and still more preferably 300 to 450 $\mu$m.

The particles of the water-absorbent resin may be in the form of particles each composed of a single particle, or particles (secondary particles) formed by agglomeration of fine particles (primary particles). Examples of the shape of the primary particles include a substantially spherical shape, a crushed indefinite shape, and a flat shape. When the particles of the water-absorbent resin are primary particles produced by reversed phase suspension polymerization, a water-absorbent resin having a substantially spherical single-particle shape with a smooth surface shape, such as a spherical shape or an elliptical shape, can be prepared. Because the primary particles having such a shape have a smooth surface shape, they have high flowability as a powder, and moreover, the agglomerated particles tend to be closely packed, and hence, the particles are unlikely to be broken even when subjected to an impact. Thus, a water-absorbent resin having a high particle strength tends to be obtained.

The median particle diameter of the water-absorbent resin can be measured using JIS standard sieves. Specifically, the median particle diameter represents a value as measured using the method described in the Examples.

The water-absorbent resin may contain additives suitable for its purpose. Examples of such additives include inorganic powders, surfactants, oxidizing agents, reducing agents, metal chelating agents, radical chain inhibitors, antioxidants, anti-bacterial agents, and deodorizers. For example, when 0.05 to 5 parts by mass of amorphous silica as an inorganic powder is added to 100 parts by mass of the water-absorbent resin, the flowability of the water-absorbent resin can be further improved.

As the method of polymerizing the water-soluble ethylenically unsaturated monomer, a representative polymerization method such as aqueous solution polymerization, emulsion polymerization, or reversed phase suspension polymerization is used. In aqueous solution polymerization, polymerization is performed by heating, optionally with stirring, an aqueous solution of the water-soluble ethylenically unsaturated monomer. In reversed phase suspension polymerization, polymerization is performed by heating the water-soluble ethylenically unsaturated monomer with stirring in a hydrocarbon dispersion medium. In the present invention, reversed phase suspension polymerization is preferred from the viewpoint of allowing precise control of the polymerization reaction and control of a wide range of particle diameters.

One exemplary method for producing the water-absorbent resin will be hereinafter described.

Specific examples of the method for producing the water-absorbent resin include a method for producing the water-absorbent resin by performing reversed phase suspension polymerization of the water-soluble ethylenically unsaturated monomer in a hydrocarbon dispersion medium, the method including the steps of performing the polymerization in the presence of a radical polymerization initiator; and post-crosslinking a hydrous gel obtained by the polymerization, in the presence of a post-crosslinking agent. In the method for producing the water-absorbent resin of the present invention, an internal-crosslinking agent may be added, as required, to the water-soluble ethylenically unsaturated monomer to obtain a hydrous gel having an internal-crosslinked structure.

<Polymerization Step>

[Water-Soluble Ethylenically Unsaturated Monomer]

Examples of the water-soluble ethylenically unsaturated monomer include (meth)acrylic acid ("acryl" and "methacryl" are herein collectively referred to as "(meth)acryl"; the same applies below) and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, from the viewpoint of being readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resins. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer is preferably dispersed as an aqueous solution in a hydrocarbon dispersion medium, and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be neutralized with an alkaline neutralizing agent, as required, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization operation. The above-mentioned alkaline neutralizing agents may be used alone or in combinations of two or more.

The degree of neutralization of the water-soluble ethylenically unsaturated monomer with an alkaline neutralizing agent, calculated as the degree of neutralization of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

[Radical Polymerization Initiator]

Examples of the radical polymerization initiator to be added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, from the viewpoint of being readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator to be used is, for example, 0.00005 to 0.01 mol per mole of the water-soluble ethylenically unsaturated monomer, although not particularly limited thereto. When the radical polymerization initiator is used in the above-defined range of amounts, the occurrence of an abrupt polymerization reaction can be avoided, and the polymerization reaction can be completed in an appropriate period of time.

[Internal-Crosslinking Agent]

Examples of the internal-crosslinking agent include those that can crosslink the polymer of the water-soluble ethylenically unsaturated monomer to be used, for example: unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N"-triallylisocyanate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and $\alpha$-methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

The amount of the internal-crosslinking agent to be used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

[Hydrocarbon Dispersion Medium]

Examples of the hydrocarbon dispersion medium include $C_{6-8}$ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. Examples of mixtures of hydrocarbon dispersion media include commercially available products, such as Exxsol Heptane (from Exxon Mobil Corporation; containing 75 to 85% by mass of heptane and its isomeric hydrocarbons). Favorable results can also be obtained using such a mixture.

The amount of the hydrocarbon dispersion medium to be used is preferably 100 to 1500 parts by mass, and more preferably 200 to 1400 parts by mass, per 100 parts by mass of a first-stage water-soluble ethylenically unsaturated monomer, from the viewpoint of homogeneously dispersing the water-soluble ethylenically unsaturated monomer, and facilitating control of the polymerization temperature. As described below, reversed phase suspension polymerization is performed in one stage (single stage) or two or more multiple stages. The above-mentioned first-stage polymerization refers to the first-stage polymerization reaction in single-stage polymerization or multi-stage polymerization (the same applies below).

[Dispersion Stabilizer]

(Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerin fatty acid esters, and sucrose fatty acid esters are particularly preferably used, from the viewpoint of dispersion stability of the monomer. These surfactants may be used alone or in combinations of two or more.

The amount of the surfactant to be used is preferably 0.1 to 30 parts by mass, and more preferably 0.3 to 20 parts by mass, per 100 parts by mass of the first-stage water-soluble ethylenically unsaturated monomer.

(Polymeric Dispersion Agent)

A polymeric dispersion agent may be used in combination with the above-described surfactant, as the dispersion stabilizer to be used in reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, from the viewpoint of dispersion stability of the monomer. These polymeric dispersion agents may be used alone or in combinations of two or more.

The amount of the polymeric dispersion agent to be used is preferably 0.1 to 30 parts by mass, and more preferably 0.3 to 20 parts by mass, per 100 parts by mass of the first-stage water-soluble ethylenically unsaturated monomer.

[Other Components]

In the method for producing the water-absorbent resin, other components may be optionally added to the aqueous solution containing the water-soluble ethylenically unsaturated monomer, which is then subjected to reversed phase suspension polymerization. Examples of the other components include various additives such as thickeners and chain transfer agents.

By way of example, a thickener may be added to the aqueous solution containing the water-soluble ethylenically unsaturated monomer, which is then subjected to reversed phase suspension polymerization. When a thickener is thus added to adjust the viscosity of the aqueous solution, the median particle diameter obtained by reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the aqueous solution containing the water-soluble ethylenically unsaturated monomer, the larger the primary particles and/or secondary particles of the resulting particles tend to be.

[Reversed Phase Suspension Polymerization]

Reversed phase suspension polymerization is performed by, for example, dispersing an aqueous monomer solution containing the water-soluble ethylenically unsaturated monomer in a hydrocarbon dispersion medium, in the presence of a dispersion stabilizer. Here, as long as the dispersion stabilizer (a surfactant or a polymeric dispersion agent) is added before the beginning of the polymerization reaction, it may be added either before or after the addition of the aqueous monomer solution.

In particular, from the viewpoint of readily reducing the amount of the hydrocarbon dispersion medium remaining in the resulting water-absorbent resin, it is preferred to disperse the aqueous monomer solution in the hydrocarbon dispersion medium in which a polymeric dispersion agent is dispersed, and then disperse a surfactant therein, followed by polymerization.

The above-described reversed phase suspension polymerization can be performed in one stage or two or more multiple stages. From the viewpoint of enhancing productivity, reversed phase suspension polymerization is preferably performed in two or three stages.

Reversed phase suspension polymerization with two or more multiple stages may be performed as follows: the first-stage reversed phase suspension polymerization is performed; subsequently, the water-soluble ethylenically unsaturated monomer is added to the reaction mixture obtained by the first-stage polymerization reaction and mixed, and reversed phase suspension polymerization in the second and subsequent stages is performed in the same manner as in the first stage. In reversed phase suspension polymerization in each of the second and subsequent stages, in addition to the water-soluble ethylenically unsaturated monomer, a radical polymerization initiator is preferably added within the above-described range of molar ratios of each component relative to the water-soluble ethylenically unsaturated monomer, based on the amount of the water-soluble ethylenically unsaturated monomer added during reversed phase suspension polymerization in each of the second and subsequent stages. In the second and subsequent stages of polymerization, an internal-crosslinking agent may also be added, as required, to the water-soluble ethylenically unsaturated monomer.

The reaction temperature during the polymerization reaction is preferably 20 to 110°C, and more preferably 40 to 90°C, from the viewpoint of allowing the polymerization to proceed quickly to reduce the polymerization time for improved economical efficiency, and readily removing the heat of polymerization to perform the reaction smoothly.

<Post-Crosslinking Step>

Through <Polymerization Step> described above, the water-soluble ethylenically unsaturated monomer is polymerized to give a hydrous gel having an internal-crosslinked structure. This hydrous gel may be directly subjected to <Drying Step> described below, or may be post-crosslinked with a post-crosslinking agent (that is, may undergo a post-crosslinking reaction), as described in this section. The post-crosslinking reaction is preferably preformed in the presence of a post-crosslinking agent, after the polymerization of the water-soluble ethylenically unsaturated monomer. When the hydrous gel having an internal-crosslinked structure is thus subjected to the post-crosslinking reaction after the polymerization, a water-absorbent resin can be obtained in which the crosslinking density in the vicinity of the surface has been increased to improve various kinds of performance, such as the water-absorption capacity under a load.

Examples of the post-crosslinking agent include compounds having two or more reactive functional groups, for example: polyols, such as ethylene glycol, propylene glycol, 1,4-butanediol, trimethylolpropane, glycerin, polyoxyethylene glycol, polyoxypropylene glycol, and polyglycerin; polyglycidyl compounds, such as (poly)ethylene glycol triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and $\alpha$-methylepichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds, such as ethylene carbonate; and hydroxyalkylamide compounds, such as bis[N,N-di($\beta$-hydroxyethyl)]adipamide. Preferred among these post-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These post-crosslinking agents may be used alone or in combinations of two or more.

The amount of the post-crosslinking agent to be used is preferably 0.00001 to 0.01 mol, more preferably 0.00005 to 0.005 mol, and still more preferably 0.0001 to 0.002 mol, per mole of the total amount of the water-soluble ethylenically unsaturated monomer used for polymerization.

The post-crosslinking agent may be added as is or as an aqueous solution. As required, a solution of the post-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a mixture with water.

The post-crosslinking agent may be added after the polymerization reaction of the water-soluble ethylenically unsaturated monomer is substantially completed. The post-crosslinking agent is preferably added in the presence of 1 to 400 parts by mass of water, more preferably 5 to 200 parts by mass of water, still more preferably 10 to 100 parts by mass of water, and even more preferably 20 to 60 parts by mass of water, per 100 parts by mass of the water-soluble ethylenically unsaturated monomer. The amount of water herein refers to the total amount of the water contained in the reaction system and the water that is used, as required, during the addition of the post-crosslinking agent.

The reaction temperature during the post-crosslinking reaction is preferably 50 to 250°C, more preferably 60 to 180°C, still more preferably 60 to 140°C, and even more preferably 70 to 120°C. The reaction time of the post-crosslinking reaction is preferably 1 to 300 minutes, and more preferably 5 to 200 minutes.

<Drying Step>

After reversed phase suspension polymerization is performed as described above, a drying step may be performed of adding external energy, such as heat, to remove the water, the hydrocarbon dispersion medium, and the like out of the system by distillation. To remove the water in the hydrous gel after reversed phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. In this case, the temperature within the system during drying is maintained at a temperature not higher than the azeotropic temperature with the hydrocarbon dispersion medium, which is preferable from the viewpoint of preventing deterioration of the resin. Subsequently, the water and the hydrocarbon dispersion medium are distilled off to obtain particles of the water-absorbent resin. By controlling the treatment conditions for the drying step after the polymerization to adjust the amount of water to be removed, various kinds of performance of the resulting water-absorbent resin can be controlled.

In the drying step, the drying treatment by distillation may be performed under atmospheric pressure or reduced pressure. The drying treatment may also be performed in a stream of nitrogen or the like, from the viewpoint of enhancing the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the post-crosslinking step with a post-crosslinking agent is performed after the polymerization of the monomer by reversed phase suspension polymerization, the drying step by distillation is performed as described above, after the completion of the post-crosslinking step. Alternatively, the post-crosslinking step and the drying step may be performed simultaneously.

Furthermore, various additives such as chelating agents, reducing agents, oxidizing agents, anti-bacterial agents, and deodorizers may be added, as required, to the water-absorbent resin, after polymerization, during drying, or after drying.

2. Absorbent Material and Absorbent Article

The water-absorbent resin composition of the present invention constitutes an absorbent material used for hygienic materials, such as sanitary items and disposable diapers, for example, and is suitably used for an absorbent article including the absorbent material.

Herein, the absorbent material obtained using the water-absorbent resin composition of the present invention contains the water-absorbent resin composition of the present invention and hydrophilic fibers. Examples of the structure of the absorbent material include a mixed dispersion obtained by mixing the water-absorbent resin composition and the hydrophilic fibers to give a homogeneous composition; a sandwich structure in which the water-absorbent resin is sandwiched between layered hydrophilic fibers; and a structure in which the water-absorbent resin composition and the hydrophilic fibers are wrapped in tissue paper. The absorbent material may also contain other components, for example, adhesive binders such as a hot melt adhesive, an adhesive emulsion, and thermally fusible synthetic fibers for enhancing the shape retention properties of the absorbent material.

The amount of the water-absorbent resin composition contained in the absorbent material is preferably 5 to 95% by mass, more preferably 20 to 90% by mass, and still more preferably 30 to 80% by mass.

Examples of the hydrophilic fibers include cellulose fibers, such as cotton-like pulp made from wood, mechanical pulp, chemical pulp, and semi-chemical pulp; artificial cellulose fibers, such as rayon and acetate; and fibers made of synthetic resins, such as hydrophilized polyamide, polyester, and polyolefin.

The absorbent article of the present invention can be obtained when the absorbent material obtained using the water-absorbent resin composition of the present invention is held between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include nonwoven fabrics of air-through type, spunbond type, chemical bond type, needle punch type, or the like, and porous synthetic resin sheets, which are made of fibers of polyethylene, polypropylene, polyester, or the like. Examples of the liquid-impermeable sheet include synthetic resin films made of resins such as polyethylene, polypropylene, and polyvinyl chloride.

Examples

The present invention will be hereinafter described in detail by showing examples and a comparative example.

Water-absorbent resins obtained in the following examples and comparative example were evaluated by the various tests described below. Each of the testing methods for evaluation will be hereinafter described.

<Median Particle Diameter>

JIS standard sieves having mesh sizes of 850 $\mu$m, 600 $\mu$m, 500 $\mu$m, 400 $\mu$m, 300 $\mu$m, 250 $\mu$m, and 150 $\mu$m, and a receptacle were combined in this order from the top.

50 g of the water-absorbent resin was placed on the top sieve of the combined sieves, and shaken for 20 minutes with a Ro-Tap shaker to perform classification. After the classification, the particle size distribution was determined by calculating the mass of the water-absorbent resin remaining on each sieve as the mass percentage relative to the total mass. With regard to this particle size distribution, the mass percentage of the water-absorbent resin remaining on the sieve was integrated in decreasing order of particle diameter. Thereby, the relationship between the sieve mesh size and the integrated value of the mass percentage of the water-absorbent resin remaining on the sieve was plotted on logarithmic probability paper. The plots on the probability paper were connected with straight lines, and a particle diameter equivalent to 50% by mass of the integrated mass percentage was determined as the median particle diameter.

<Water Content>

About 2 g of the water-absorbent resin was precisely weighed into a previously weighed aluminum foil case (No. 8) (mass of the water-absorbent resin before drying: Wa (g)). The above-mentioned sample was dried for 2 hours in a hot-air dryer (from ADVANTEC) having an internal temperature set at 105°C. Thereafter, the sample was allowed to cool in a desiccator, and the mass Wb (g) of the water-absorbent resin after drying was measured. The water content of the water-absorbent resin was calculated from the following equation:

$$\text{Water content } (\%) = [\text{Wa} - \text{Wb}]/\text{Wa} \times 100$$

<Ammonia Generation Inhibition Test>

Artificial urine was prepared by dissolving, in 1 kg of distilled water, 25 g of urine, 9 g of sodium chloride, 0.6 g of magnesium sulfate (7-hydrate), 0.7 g of calcium lactate, 4 g of potassium sulfate, and 2.5 g of ammonium sulfate. Additionally, 1.0 mL of urease (from MERCK; 1000 U/mL *Canavalia ensiformis-derived* 50% glycerin solution) was diluted 500-fold in distilled water to prepare a urease solution. 1 g of each of the samples (the water-absorbent resin compositions of Examples 1 to 6 and the water-absorbent resin of Comparative Example 1) was placed in an antimicrobial petri dish, urease-containing artificial urine (prepared by mixing 30 g of the artificial urine and 1 mL of the urease solution) was added thereto, and the sample was allowed to swell. After the addition of the urease-containing artificial urine, the sample was sealed in a 2 L Tedlar bag, the air within the bag was removed, and 900 mL of dry air instead was added into the bag. Next, the sample was stored at 30°C, and 24 hours after, the reading from a gas detector tube (from Gastec Corporation; Ammonia 3L, 3La, and 3M) was recorded. The results are shown in Table 1, relative to the measurement value (200 ppm) as 100% (the amount of ammonia generation of Comparative Example 1).

<Flowability Test>

Flowability was evaluated for the water-absorbent resin compositions of Examples 3, 4, and 6 as representative examples and the water-absorbent resin of Comparative Example 1, by measuring the angle of spatula using the powder tester PT-N (from Hosokawa Micron Corporation). The angle of spatula refers to the angle of inclination of a side surface of a resin powder deposited on a spatula; it refers to the angle required to cause movement of the powder in a stationary state; and it is one of the items that represent the index of flowability. A smaller angle of spatula means better flowability. The measurement was performed in accordance with the procedure described in the manual of the powder tester. The measurement values of the angle of spatula are shown in Table 2.

<Production of Water-Absorbent Resin>

(Production Example 1)

A 2 L cylindrical round-bottomed separable flask having a stirrer, two sets of paddle blades, a reflux condenser, a dropping funnel, and a nitrogen gas inlet tube was prepared. 340 g of n-heptane was placed in this flask, and then 0.92 g of sucrose stearate (from Mitsubishi-Kagaku Foods Corporation; Ryoto sugar ester S-370) and 0.92 g of a maleic anhydride modified ethylene-propylene copolymer (from Mitsui Chemicals, Inc.; Hi-wax 1105A) were added thereto. The mixture was heated to 80°C with stirring to dissolve the surfactant, and then cooled to 50°C.

Separately, 92 g (1.02 mol) of an 80% by mass aqueous solution of acrylic acid was placed in a 500 mL Erlenmeyer flask, and 146.0 g of a 21% by mass aqueous solution of sodium hydroxide was added dropwise, with external cooling, to accomplish 75 mol% neutralization. Then, 0.11 g (0.41 mmol) of potassium persulfate as a radical polymerization initiator and 8.10 mg (0.046 mmol) of ethylene glycol diglycidyl ether as an internal-crosslinking agent were added and dissolved. As a result, a first-stage aqueous monomer solution was prepared.

The entire amount of the first-stage aqueous monomer solution was added to the separable flask, and the atmosphere within the system was sufficiently replaced with nitrogen. Thereafter, the flask was immersed in a water bath at 70°C and heated, and first-stage polymerization was performed for 30 minutes. As a result, a first-stage reaction mixture was obtained.

Separately, 128.8 g (1.43 mol) of an 80% by mass aqueous solution of acrylic acid was placed in another 500 mL Erlenmeyer flask, and 159.0 g of a 27% by mass aqueous solution of sodium hydroxide was added dropwise, with external cooling, to accomplish 75 mol% neutralization. Then, 0.16 g (0.59 mmol) of potassium persulfate as a radical polymerization initiator and 6.62 mg (0.038 mmol) of ethylene glycol diglycidyl ether as an internal-crosslinking agent were added and dissolved. As a result, a second-stage aqueous monomer solution was prepared.

The first-stage reaction mixture was cooled to 28°C, and the second-stage aqueous monomer solution at the same temperature was added into the system. The mixture was held at 25°C for 30 minutes while replacing the atmosphere within the system with nitrogen. Thereafter, the flask was again immersed in a water bath at 70°C and heated, and second-stage polymerization was performed for 30 minutes.

After the second-stage polymerization, the reaction mixture was heated in an oil bath at 125°C to distill 248 g of water out of the system while refluxing n-heptane by azeotropic distillation of water and n-heptane. Then, 4.42 g (0.51 mmol) of a 2% by mass aqueous solution of ethylene glycol diglycidyl ether was added, and the post-crosslinking reaction was performed at 80°C for 2 hours. Thereafter, the reaction mixture was heated in an oil bath at 125°C to evaporate n-heptane, and dried to obtain 230.5 g of a water-absorbent resin. The water-absorbent resin had a median particle diameter of 400 $\mu$m and a water content of 5.1%.

<*Onagraceae* Family Plant Extract>

As commercially available products of *Onagraceae* family plant extracts, trade name "Evening Primrose Extract-WSPS, *Oenothera biennis* extract content 100%, powder" from Oryza Oil & Fat Chemical Co., Ltd. and trade name "LUNAWHITE B, *Oenothera biennis* extract content 1% solution" from Ichimaru Pharcos Co., Ltd. were prepared.

(Example 1)

To the water-absorbent resin obtained in Production Example 1, trade name "Evening Primrose Extract-WSPS, *Oenothera biennis* extract content 100%, powder" from Oryza Oil & Fat Chemical Co., Ltd. as an *Onagraceae* family plant extract was added in an amount of 0.05 part by mass per 100 parts by mass of the water-absorbent resin, and mixed to produce a water-absorbent resin composition.

(Example 2)

A water-absorbent resin composition was produced as in Example 1, except that the amount of the *Onagraceae* family plant extract added was 0.1 part by mass per 100 parts by mass of the water-absorbent resin.

(Example 3)

A water-absorbent resin composition was produced as in Example 1, except that the amount of the *Onagraceae* family plant extract added was 0.5 part by mass per 100 parts by mass of the water-absorbent resin.

(Example 4)

A water-absorbent resin composition was produced as in Example 1, except that the amount of the *Onagraceae* family plant extract added was 1.0 part by mass per 100 parts by mass of the water-absorbent resin.

(Example 5)

A water-absorbent resin composition was produced as in Example 1, except that the amount of the *Onagraceae* family plant extract added was 2.5 parts by mass per 100 parts by mass of the water-absorbent resin.

(Example 6)

To the water-absorbent resin obtained in Production Example 1, trade name "LUNAWHITE B, *Oenothera biennis* extract content 1% solution" from Ichimaru Pharcos Co., Ltd. as an *Onagraceae* family plant extract was added in an amount of 0.5 part by mass (content ratio) per 100 parts by mass of the water-absorbent resin, and the mixture was heated to remove the solvent to produce a water-absorbent resin composition. More specifically, in order to homogeneously mix the *Onagraceae* family plant extract with the water-absorbent resin, a solution of a mixture of 5 g of the *Onagraceae* family plant extract and 5 g of ethanol was placed in a 50 mL beaker, and then 10 g of the water-absorbent resin obtained in Production Example 1 was added with stirring using a stirrer bar having a size of 0.7 cm × 2.0 cm. After stirring was continued for 20 minutes, the stirrer bar was removed, and the contents within the beaker were spread in a stainless steel petri dish having an inside diameter of 10 cm, and dried under reduced pressure at 80°C for 8 hours to produce a water-absorbent resin composition.

(Comparative Example 1)

The water-absorbent resin produced in Production Example 1 was used as is, as the water-absorbent resin of Comparative Example 1.

[Table 1]

| | *Onagraceae* family plant | | | Amount (%) of ammonia generation after 24 hours (relative to the amount in Comparative Example 1 as 100%) |
|---|---|---|---|---|
| | Type | State | Amount added (part(s) by mass) | |
| Comparative Example 1 | - | - | - | 100 |
| Example 1 | *Oenothera biennis* | Powder | 0.05 | 50 |
| Example 2 | | Powder | 0.1 | 23 |
| Example 3 | | Powder | 0.5 | 1 |
| Example 4 | | Powder | 1.0 | 4 |
| Example 5 | | Powder | 2.5 | 3 |
| Example 6 | | Liquid | 0.5 | 5 |

[Table 2]

|  | State | Flowability test (angle (degree) of spatula) |
|---|---|---|
| Comparative Example 1 | - | 48.2 |
| Example 3 | Powder | 30.1 |
| Example 4 | Powder | 28.4 |
| Example 6 | Liquid | 30.9 |

**Claims**

1. A water-absorbent resin composition comprising an *Onagraceae* family plant extract and a water-absorbent resin, wherein

   the *Onagraceae* family plant extract is contained in an amount of 0.05 parts by mass or more per 100 parts by mass of the water-absorbent resin,
   the *Onagraceae* family plant extract is an *Oenothera* genus plant extract,
   the *Onagraceae* family plant extract is obtained from seeds using water or hydrous ethanol,
   the water-absorbent resin is in the form of particles, wherein the water-absorbent resin composition is produced by mixing the *Onagraceae* family plant extract and the water-absorbent resin.

2. The water-absorbent resin composition according to claim 1, wherein the *Onagraceae* family plant extract is contained in an amount of 0.05 to 5 parts by mass per 100 parts by mass of the water-absorbent resin.

3. An absorbent material comprising the water-absorbent resin composition according to claim 1 or 2, and a hydrophilic fiber.

4. An absorbent article comprising the absorbent material according to claim 3, the absorbent material being held between a liquid-permeable sheet and a liquid-impermeable sheet.

**Patentansprüche**

1. Wasserabsorbierende Harzzusammensetzung, die einen Pflanzenextrakt der Onagraceae-Familie und ein wasserabsorbierendes Harz umfasst, worin

   der Pflanzenextrakt der Onagraceae-Familie in einer Menge von 0,05 Massenteilen oder mehr pro 100 Massenteile des wasserabsorbierenden Harzes enthalten ist,
   der Pflanzenextrakt der Onagraceae-Familie ein Pflanzenextrakt der Gattung *Oenothera* ist,
   der Pflanzenextrakt der *Onagraceae*-Familie unter Verwendung von Wasser oder wässrigem Ethanol erhalten wurde,
   das wasserabsorbierende Harz in Form von Teilchen vorliegt, wobei die wasserabsorbierende Harzzusammensetzung durch Vermischen des Pflanzenextrakts der Onagraceae-Familie und des wasserabsorbierenden Harzes hergestellt wurde.

2. Wasserabsorbierende Harzzusammensetzung nach Anspruch 1, worin der Pflanzenextrakt der Onagraceae-Familie in einer Menge von 0,05 Massenteilen bis 5 Massenteilen pro 100 Massenteile des wasserabsorbierenden Harzes enthalten ist.

3. Absorbierendes Material, das eine wasserabsorbierende Harzzusammensetzung nach Anspruch 1 oder 2 und eine hydrophile Faser umfasst.

4. Absorbierender Gegenstand, der ein absorbierendes Material nach Anspruch 3 umfasst, wobei das absorbierende Material zwischen einer flüssigkeitsdurchlässigen Lage und einer flüssigkeitsundurchlässigen Lage gehalten wird.

**Revendications**

1. Composition de résine absorbant l'eau comprenant un extrait d'une plante appartenant à la famille des *Onagracées* et une résine absorbant l'eau, dans laquelle

   l'extrait de la plante appartenant à la famille des *Onagracées* est contenu en une quantité de 0,05 partie en masse ou plus par 100 parties en masse de la résine absorbant l'eau,
   l'extrait de la plante appartenant à la famille des *Onagracées* est un extrait d'une plante du genre *Oenothera,*
   l'extrait de la plante appartenant à la famille des *Onagracées* est obtenu à partir de graines en utilisant de l'eau ou de l'éthanol aqueux,
   la résine absorbant l'eau est sous la forme de particules, dans laquelle la composition de résine absorbant l'eau est produite en mélangeant l'extrait de la plante appartenant à la famille des *Onagracées* et la résine absorbant l'eau.

2. Composition de résine absorbant l'eau selon la revendication 1, dans laquelle l'extrait de la plante appartenant à la famille des *Onagracées* est contenu en une quantité de 0,05 à 5 parties en masse par 100 parties en masse de la résine absorbant l'eau.

3. Matériau absorbant comprenant la composition de résine absorbant l'eau selon la revendication 1 ou 2, et une fibre hydrophile.

4. Article absorbant comprenant le matériau absorbant selon la revendication 3, le matériau absorbant étant maintenu entre une feuille perméable aux liquides et une feuille imperméable aux liquides.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104173591 A
- KR 20100039532 A
- JP 2001037805 A
- JP H11512946 A
- JP H651843 B
- JP 2001505237 A